(19) Europäisches Patentamt
European Patent Office
Office européen des brevets

(11) EP 4 151 718 A1

(12) EUROPEAN PATENT APPLICATION
published in accordance with Art. 153(4) EPC

(43) Date of publication:
**22.03.2023 Bulletin 2023/12**

(21) Application number: **21804471.7**

(22) Date of filing: **11.05.2021**

(51) International Patent Classification (IPC):
***C12N 1/21*** *(2006.01)* ***C12N 15/31*** *(2006.01)*
***C12P 7/40*** *(2006.01)* ***C12P 7/42*** *(2006.01)*
***C12P 7/44*** *(2006.01)* ***C12P 7/46*** *(2006.01)*
***C12P 7/54*** *(2006.01)*

(52) Cooperative Patent Classification (CPC):
**C07K 14/195; C12N 15/74; C12P 7/40; C12P 7/42; C12P 7/44; C12P 7/46; C12P 7/54**

(86) International application number:
**PCT/JP2021/017802**

(87) International publication number:
**WO 2021/230222 (18.11.2021 Gazette 2021/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.05.2020 JP 2020083660**

(71) Applicant: **Toray Industries, Inc.**
**Tokyo 103-8666 (JP)**

(72) Inventors:
- **NAKAMURA, Hitomi**
  **Kamakura-shi, Kanagawa 248-8555 (JP)**
- **ISOBE, Kyohei**
  **Kamakura-shi, Kanagawa 248-8555 (JP)**
- **KAWAMURA, Kenji**
  **Kamakura-shi, Kanagawa 248-8555 (JP)**
- **YAMADA, Katsushige**
  **Kamakura-shi, Kanagawa 248-8555 (JP)**

(74) Representative: **Kador & Partner PartG mbB**
**Corneliusstraße 15**
**80469 München (DE)**

Remarks:
The complete document including Reference Table(s) and the Sequence Listing(s) can be downloaded from the EPO website

# (54) GENETICALLY MODIFIED MICROORGANISM AND METHOD FOR PRODUCING ORGANIC ACID

(57) A genetically modified microorganism containing a gene capable of expressing a mutated YeeX protein or a homolog thereof, which results from substitution, insertion, and/or deletion of one to several amino acids in the amino acid sequence of the wild-type YeeX protein or of a homolog thereof, has a higher capacity to produce an organic acid(s) than the genetically unmodified original microorganism.

EP 4 151 718 A1

## Description

### TECHNICAL FIELD

The present invention relates to a genetically modified microorganism capable of producing an organic acid at a high yield and to a method of producing an organic acid by using the genetically modified microorganism.

### BACKGROUND ART

Organic acids produced by using fermentative production processes by microorganisms are widely used in industries. For example, succinic acid is used in various products, such as pharmaceutical products, food additives, and bath powders, and acetic acid is widely used as foods and chemical reagents. As a method of producing an organic acid including succinic acid or acetic acid by using a microorganism, a method using a modified strain of a microorganism is disclosed in Patent Document 1, in which the expression of at least one gene selected from the group consisting of genes encoding phosphoglycerate kinase, phosphofructokinase, glyceraldehyde-3-phosphate dehydrogenase, and fumarate hydratase, genes encoding proteins of the phosphotransferase system, and genes encoding oxidative stress-responsive factors is increased compared with that in the unmodified strain of the microorganism to reduce the effect of fermentation inhibitors in a sugar solution obtained from inedible resources when the sugar solution is used as a raw material for fermentation.

Moreover, industries are also paying attention to 3-hydroxyadipic acid (IUPAC name: 3-hydroxyhexanedioic acid), α-hydromuconic acid (IUPAC name: (E)-hex-2-enedioic acid), and adipic acid (IUPAC name: hexanedioic acid), which are dicarboxylic acids containing six carbon atoms. Each of the dicarboxylic acids can be polymerized with a polyvalent amine and can be used as a raw material for polyamide resins. Additionally, these dicarboxylic acids can be used alone after ammonia addition at a terminal position in these chemicals to form lactams as raw materials for polyamide production. As a method of producing 3-hydroxyadipic acid or the like, a genetically modified microorganism and a method of the production of the substances using the microorganism are disclosed in Patent Document 2, in which a nucleic acid encoding a polypeptide involved in the production of 3-hydroxyadipic acid and α-hydromuconic acid has been introduced or the expression of the polypeptide is increased in the genetically modified microorganism.

On the other hand, YeeX protein is classified as a DUF496 family protein. Although little information is known about YeeX protein, Non-Patent Document 1 has demonstrated the expression of YeeX protein in *Escherichia coli* by two-dimensional SDS-PAGE analysis. Patent Document 3 describes YeeX protein as an example of a protein that helps protein production. However, none of the documents describe the effect of YeeX protein on the production of organic acids.

### PRIOR ART DOCUMENTS

#### Patent Documents

Patent Document 1: JP-A 2017-192325
Patent Document 2: WO2019/107516
Patent Document 3: US2007/0298418

#### Non-Patent Document

Non-Patent Document 1: FEMS Microbiology Letters, vol. 169, p. 375-382 (1998).

### SUMMARY OF THE INVENTION

#### Problems to be Solved by the Invention

An object of the present invention is to promote the yields of the products in the methods of producing chemicals represented by organic acids using a microorganism. Specifically, the object is to increase the ability of the microorganisms to produce the chemicals represented by organic acids, by introducing a mutation(s) into the microorganisms.

#### Means for Solving the Problem

The present inventors intensively studied for attaining the above object, focusing on a gene encoding YeeX

protein, whose function had been unknown, to discover that a microorganism comprising a gene capable of expressing a mutated YeeX protein or a homolog thereof has a higher capacity to produce a chemical(s) represented by an organic acid(s), thereby reaching the present invention.

Specifically, the present invention is constituted by the following (1) to (10).

(1) A genetically modified microorganism comprising a gene capable of expressing a mutated YeeX protein or a homolog thereof, which results from substitution, insertion, and/or deletion of one to several amino acids in the amino acid sequence of the wild-type YeeX protein or of a homolog thereof.

(2) The genetically modified microorganism according to (1), wherein the mutated YeeX protein or a homolog thereof is a mutated YeeX protein or a homolog thereof, which results from substitution, insertion, and/or deletion of one to several amino acids within a region corresponding to the amino acid residues 74 to 100 in the amino acid sequence of SEQ ID NO: 1.

(3) The genetically modified microorganism according to (1) or (2), wherein the mutated YeeX protein or a homolog thereof is a mutated YeeX protein or a homolog thereof with a mutation, which is a substitution of the alanine corresponding to the amino acid at position 84 in the amino acid sequence of SEQ ID NO: 1 to another amino acid.

(4) The genetically modified microorganism according to any one of (1) to (3), wherein the gene capable of expressing the mutated YeeX protein or a homolog thereof is present in the genome.

(5) The genetically modified microorganism according to (4), which results from mutation or replacement of a gene expressing the wild-type YeeX protein or a homolog thereof in the genome into or with the gene capable of expressing the mutated YeeX protein or a homolog thereof.

(6) The genetically modified microorganism according to any one of (1) to (5), wherein the mutated YeeX protein or a homolog thereof has a mutation, which is a substitution of the alanine corresponding to the amino acid at position 84 in the amino acid sequence of SEQ ID NO: 1 to valine.

(7) The genetically modified microorganism according to any one of (1) to (6), wherein the microorganism has an ability to produce an organic acid.

(8) The genetically modified microorganism according to any one of (1) to (7), wherein the microorganism is a microorganism belonging to the genus *Serratia, Escherichia, Actinobacillus, Basfia, Pseudomonas, Hafnia, Acinetobacter, Shimwellia,* or *Aerobacter.*

(9) A method of producing an organic acid(s), comprising culturing the genetically modified microorganism according to (7) or (8) in a culture medium containing a carbon source as a raw material for fermentation.

(10) The method of producing an organic acid(s) according to (9), wherein the organic acid(s) is/are succinic acid, acetic acid, 3-hydroxyadipic acid, $\alpha$-hydromuconic acid, and/or adipic acid.

## Effects of the Invention

The presence of the gene capable of expressing the mutated YeeX protein or a homolog thereof allows the genetically modified microorganism according to the present invention to produce a higher yield(s) of a chemical(s) represented by an organic acid(s), than the genetically unmodified original microorganism.

## DETAILED DESCRIPTION OF THE INVENTION

The present invention will be described below in more details but is not limited to the following embodiments and can be practiced with various modifications within the gist of the present invention.

The genetically modified microorganism of the present invention is characterized by the presence of a gene capable of expressing a mutated YeeX protein or a homolog thereof. The method of the present invention to produce an organic acid(s) is characterized by culturing the genetically modified microorganism.

YeeX protein is a protein belonging to the DUF496 family. The symbol "*yeerY*" refers to a gene encoding YeeX protein. YeeX protein has been suggested to contain a structure similar to that of transcription factors from an HHPred (homology detection & structure prediction by HMM-HMM comparison) search result, which was based on the prediction of a secondary structure from the amino acid sequence of SEQ ID NO: 1.

The homolog of YeeX protein is a wild-type protein that shows a high sequence identity to the amino acid sequence of a protein identified as YeeX protein and is predicted to have a similar function or structure to YeeX protein. YeeX protein used in the present invention preferably has a sequence identity of 50% or more, more preferably 55% or more, still more preferably 70% or more, yet more preferably 80% or more, yet more preferably 90% or more, particularly preferably 95% or more, to the amino acid sequence of SEQ ID NO: 1.

Examples of YeeX protein or a homolog thereof include YeeX protein from *Escherichia coli* (NCBI Protein ID: NP_416511, SEQ ID NO: 1), a homolog of YeeX protein from *Serratia grimesii* (NCBI Protein ID: HCJ99940, SEQ ID NO: 2), a homolog of YeeX protein from *Acinetobacter baumanii* (NCBI Protein ID: AAL09094, SEQ ID NO: 3), a homolog

of YeeX protein from *Actinobacillus succinogenes* (NCBI Protein ID: WP_012072666, SEQ ID NO: 4), a homolog of YeeX protein from *Aerobacter cloacae* (NCBI Protein ID: WP_115875767, SEQ ID NO: 5), a homolog of YeeX protein from *Basfia succiniciproducens* (Protein ID: WP 011200744, SEQ ID NO: 6), a homolog of YeeX protein from *Hafnia paralvei* (Protein ID: WP_004089583, SEQ ID NO: 7), a homolog of YeeX protein from *Pseudomonas aeruginosa* (Protein ID: MXH34489, SEQ ID NO: 8), and a homolog of YeeX protein from *Shimwellia blattae* (Protein ID: WP_002439990, SEQ ID NO: 9).

In the present invention, the term "sequence identity" means a ratio (percentage) of the number of identical amino acid or nucleotide residues relative to the total number of amino acid or nucleotide residues over the overlapping portion of an amino acid sequence alignment (including an amino acid corresponding to the translation start site) or a nucleotide sequence alignment (including the start codon), which is obtained by aligning two amino acid sequences or nucleotide sequences with or without introduction of a gap(s) for an optimal match, and is calculated by the following formula (1). The sequence identity can be easily determined using BLAST (Basic Local Alignment Search Tool), an algorithm widely used in this field. For example, BLAST is publicly available on a website, such as that of NCBI (National Center for Biotechnology Information) or KEGG (Kyoto Encyclopedia of Genes and Genomes), on which the sequence identity can be easily determined using default parameters.

Sequence identity (%) = the number of matches (gap matches are ignored) / the length of the shorter sequence (the sequence length excluding gaps) × 100 ... Formula (1)

By using a function of Genetyx (% Identity Matrix) to calculate sequence identities based on the formula (1) among the amino acid sequences of SEQ ID NOs: 1 to 9, the lowest sequence identity of 54.46% is found between the sequences of SEQ ID NOs: 4 and 9, and thus the sequence identities among the amino acid sequences of SEQ ID NOs: 1 to 9 are found to be at least 50% or more. The results of calculation of sequence identity using Genetyx are presented in Table 1. In Table 1 below, the numbers in the leftmost column are the SEQ ID NOs of the indicated sequences.

[Table 1]

[GENETYX : % Identity Matrix]
*Gaps are NOT taken into account.
[%]

| Shimwel | 1. Escheri | 2. Serrati | 3.. Acineto | 4. Actinob | 5. Aerobac | 6. Basfia | 7. Hafnia | 8. Pseudom |
|---|---|---|---|---|---|---|---|---|
| 1. Escherichia coli | * | | | | | | | |
| 2. *Serratia* grimesii | 78.50 | * | | | | | | |
| 3. Acinetobacter *baumanii* | 90.42 | 76.59 | * | | | | | |
| 4. Actinobacillus succinogenes | 56.88 | 57.94 | 55.31 | * | | | | |
| . Aerobacter cloacae | 97.24 | 78.50 | 90.42 | 55.85 | * | | | |
| 6. Basfia | 60.55 | 64.48 | 57.44 | 68.42 | 58.55 | * | | |
| 7. Hafnia | 78.50 | 82.24 | 76.59 | 58.87 | 76.63 | 65.42 | | |
| 9. Pseudomonas aeruginosa | 93.45 | 77.57 | 87.23 | 56.07 | 95.32 | £i:>..δ: | 75.70 | * |
| 9. Shimwellia blattae | 92.66 | 77.57 | 90.42 | 54.46 | 91.89 | 56.25 | 75.70 | 93.45 |
| * | | | | | | | | |

(continued)

| [Match Count/Length] | 1. Escheri | 2. Serrati | 3. Acineto | 4. Actinob | 5. Aerobac | 6. Basfia | 7. Hafnia | 8. Pseudom | Shimwel |
|---|---|---|---|---|---|---|---|---|---|
| 1. Escherichia. coli | * | | | | | | | | |
| 2. Serratia grimesii | 84/107 | * | | | | | | | |
| 3. Acinetobacter *baumanii* | 85/94 | 72/94 | * | | | | | | |
| 4. Actinobacillus succinogenes | 62/109 | 62/107 | 52/94 | * | | | | | |
| 5. Aerobacter cloacae | 106/109 | 84/107 | 85/94 | 62/111 | * | | | | |
| 6. Basfia | 66/109 | 69/107 | 54/94 | 78/114 | 65/111 | * | | | |
| 7. Hafnia | 84/107 | 88/107 | 72/94 | 63/107 | 82/107 | 70/107 | * | | |
| 8. Pseudomonas aeruginosa | 100/107 | 83/107 | 82/94 | 60/107 | 102/107 | 67/107 | 81/107 | * | |
| 9. Shimwellia blattae | 101/109 | 83/107 | 85/94 | 61/112 | 102/111 | 63/112 | 31/107 | 100/107 | * |

The gene encoding YecX protein or a homolog thereof is not specifically limited, provided that the gene has a nucleotide sequence that can be translated into any of the amino acid sequences of SEQ ID NOs: 1 to 9 or into an amino acid sequence homologous to these amino acid sequences, and the nucleotide sequence can be determined considering the set of codons (standard genetic code) corresponding to each amino acid. In this case, the nucleotide sequences may be redesigned using codons that are frequently used by the host microorganism used in the present invention.

Specific examples of the nucleotide sequences of genes encoding polypeptides with the amino acid sequences of SEQ ID NOs: 1 and 2 include the nucleotide sequences of SEQ ID NOs: 10 and 11, respectively.

The region of the amino acid residues 74 to 100 in the amino acid sequence of SEQ ID NO: 1 is predicted to form an $\alpha$-helix structure, so that the region has a high sequence identity among different species, which is one of the features shared by YeeX protein and homologs thereof. The secondary structure of a protein can be easily identified by using a well-known web application such as Quick2D to analyze the amino acid sequence of the protein. One of the features shared by YeeX protein and homologs thereof is that the amino acid residue at position 84 in the amino acid sequence of SEQ ID NO: 1 is alanine. A multiple alignment of the amino acid sequences of SEQ ID NOs: 1 to 9 shown in Table 2, which are the amino acid sequences of YeeX protein and homologs thereof, indicates that a region corresponding to the amino acid residues 74 to 100 in the amino acid sequence of SEQ ID NO: 1, within which an $\alpha$-helix structure is predicted to form, and an alanine corresponding to the amino acid residue at position 84, are conserved.

[Table 2]

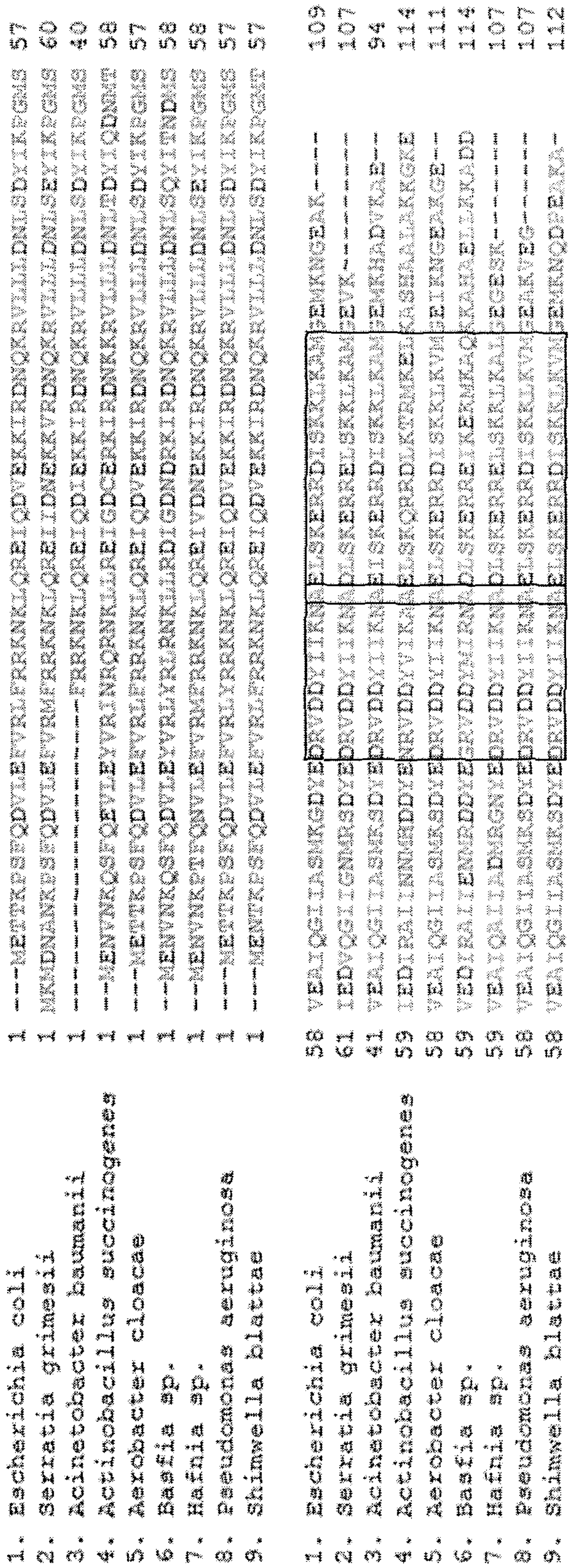

The mutated YeeX protein or a homolog thereof in the present invention is characterized by the presence of a mutation(s) caused by substitution, insertion and/or deletion of one to several amino acids, specifically one to ten amino acids, preferably one to five amino acids, more preferably one to three amino acids, still more preferably one to three

amino acids, yet more preferably one or two amino acids, particularly preferably one amino acid, in the amino acid sequence of the wild-type YeeX protein or of a homolog thereof. The mutation(s) is/are not limited to specific position(s) but is/are preferably located within a region corresponding to the amino acid residues 74 to 100, a region showing a high sequence identity among different species and predicted to form an α-helix structure, more preferably within a region corresponding to the amino acid residues 74 to 94, still more preferably within a region corresponding to the amino acid residues 75 to 88, yet more preferably within a region corresponding to the amino acid residues 82 to 88, and particularly preferably at the alanine corresponding to the amino acid residue at position 84 in the amino acid sequence of SEQ ID NO: 1.

In cases where a mutated site in the mutated YeeX protein or a homolog thereof is located at the alanine corresponding to the amino acid residue at position 84 in the amino acid sequence of SEQ ID NO: 1, the mutation is preferably caused by a substitution of the alanine residue with an amino acid other than alanine. The amino acid other than alanine refers to any amino acid residue and preferably to a hydrophobic amino acid residue such as valine, leucine, isoleucine, proline, glycine, methionine, or phenylalanine, more preferably to valine, leucine, or isoleucine, and still more preferably to valine.

Expression of a mutated YeeX protein or a homolog thereof in the genetically modified microorganism of the present invention is not limited to a specific method. Specific examples of the method include a method for introduction of a mutation(s) into an endogenous gene encoding YeeX protein or a homolog thereof by a known technique; a method for introduction of a gene encoding YeeX protein or a homolog thereof by using an expression vector autonomously replicable in a microorganism; a method for introduction of a gene encoding a mutated YeeX protein or a homolog thereof into the genome of a microorganism by using a technique such as homologous recombination; and a method for replacement of an endogenous gene encoding YeeX protein or a homolog thereof with a gene encoding a mutated YeeX protein or a homolog thereof. For the genetically modified microorganism of the present invention, preferred methods include a method for integrating a gene of interest into an expression vector autonomously replicable in a microorganism and then introducing the resulting expression vector into the microorganism; and a method for placing a gene encoding a mutated YeeX protein or a homolog thereof into the genome of a host microorganism by introducing a mutation(s) into an endogenous gene encoding YeeX protein or a homolog thereof, or by replacing the gene of interest with a gene encoding a mutated YeeX protein or a homolog thereof by a technique such as homologous recombination.

The microorganism used in the present invention is not limited to a specific microorganism as long as the microorganism is a genetically modified microorganism comprising a gene encoding a mutated YeeX protein or a homolog of the mutated YeeX protein, and the microorganism is preferably a microorganism capable of producing a chemical(s), more preferably capable of producing an organic acid(s) or an amino acid(s), and still more preferably capable of producing an organic acid(s). Specifically, the microorganism is preferably a microorganism selected from the group consisting of the genera *Serratia, Escherichia, Actinobacillus, Basfia, Pseudomonas, Hafnia, Acinetobacter, Shimwellia,* and *Aerobacter,* more preferably a microorganism selected from the group consisting of the genera *Serratia, Escherichia, Actinobacillus,* and *Basfia,* and particularly preferably a microorganism belonging to the genus *Serratia* or *Escherichia.*

In cases where a genetically modified microorganism of the present invention has an ability to produce an organic acid, the genetically modified microorganism is characterized by a higher capacity to produce the organic acid than the genetically unmodified original microorganism lacking the gene capable of expressing a mutated YeeX protein or a homolog thereof. The phrase "higher capacity to produce an organic acid" refers to a higher yield of the organic acid produced by the genetically modified microorganism producing under the same fermentation conditions than by a microorganism strain comprising a gene capable of expressing only the wild-type YeeX protein of SEQ ID NO: 1 or than by a microorganism strain lacking a gene capable of expressing YeeX protein, where these microorganisms are all derived from the same host microorganism. In the method of organic acid production using the genetically modified microorganism of the present invention, the yield of acetic acid is calculated according to the formula (2). The yield of succinic acid, 3-hydroxyadipic acid, α-hydromuconic acid, or adipic acid is calculated according to the formula (2) modified by replacing acetic acid with succinic acid, 3-hydroxyadipic acid, α-hydromuconic acid, or adipic acid, respectively.

$$\text{Yield (\%)} = \text{Acetic acid (mol)} / \text{carbon source consumption (mol)} \times 100 \quad \ldots \quad \text{Formula (2)}$$

In cases where a gene encoding a mutated YeeX protein to be expressed in the present invention is integrated into an expression vector, it is preferable that the expression vector be constituted by a promoter, a ribosome-binding site, the gene encoding the protein to be expressed, and a transcription termination sequence.

In cases where a gene encoding a mutated YeeX protein is integrated into the genome of a microorganism, it is preferable that a nucleic acid to be integrated into the genome be constituted by a promoter, a ribosome-binding site, the gene encoding the protein to be expressed, and a transcription termination sequence, or be integrated such that a gene encoding the wild-type YeeX protein or a homolog thereof naturally present in the microorganism is replaced by

the gene encoding the mutated YeeX protein or a homolog thereof. A gene that controls the activity of the promoter may be contained in the nucleic acid.

The promoter used in the present invention is not specifically limited, provided that the promoter can direct expression of an enzyme in a microorganism, and examples of the promoter include *gap* promoter, *trp* promoter, *lac* promoter, *tac* promoter, and T7 promoter.

In cases where the present invention uses an expression vector for introduction of a gene or for expression of a protein, the vector is not limited to a specific expression vector as long as the expression vector is autonomously replicable in the microorganism, and examples of the expression vector include pBBR1MCS vector, pBR322 vector, pMW vector, pET vector, pRSF vector, pCDF vector, pACYC vector, and derivatives of the above vectors.

In cases where the present invention uses a genome-integrating nucleic acid for introduction of a gene or for expression of a protein, the nucleic acid is integrated into the genome by site-directed homologous recombination. The site-directed homologous recombination is not limited to a specific method, and examples of the method include a method using λ Red recombinase and *sacB* gene (Biosci. Biotechnol. Biochem. 2007; 71 (12) : 2905-2911 .), and a method using λ Red recombinase and FLP recombinase (Proc. Natl. Acad. Sci. U.S.A. 2000; 97 (12) : 6640-6645.).

The method for introduction of the expression vector or of the genome-integrating nucleic acid is not limited to a specific method as long as the method allows for introduction of a nucleic acid into the microorganism. Examples of the method include electroporation (J. Bacteriol. 1988; 170: 2796-2801.) and the calcium ion method (J. Mol. Biol. 1970; 53 (1) : 159-162.).

In cases where a genetically modified microorganism of the present invention has an ability to produce an organic acid, the organic acid produced is an organic acid that the microorganism can produce and is accumulated in a culture medium. Specific examples of the organic acid include, but are not limited to, carboxylic acids such as acetic acid, succinic acid, formic acid, pyruvic acid, fumaric acid, malic acid, oxaloacetic acid, citric acid, levulinic acid, 3-oxoadipic acid, 3-hydroxyadipic acid, α-hydromuconic acid, adipic acid, and 2,5-furandicarboxylic acid. Among these carboxylic acids, acetic acid, succinic acid, 3-hydroxyadipic acid, α-hydromuconic acid, and/or adipic acid are preferred because the effect of the present invention is clearly observed. In this Description, 3-hydroxyadipic acid may be abbreviated as 3HA, α-hydromuconic acid may be abbreviated as HMA, and adipic acid may be abbreviated as ADA.

Organic acids are produced by reaction pathways present in the genetically modified microorganism of the present invention. Particularly, 3HA, HMA, and ADA are produced by a reaction pathway shown in the following Scheme 1, and a microorganism strain that expresses enzymes catalyzing the reaction A, reaction B, reaction C, reaction D, reaction E, reaction F, and reaction G is used for the production of the organic acids by fermentation.

[Chem 1]

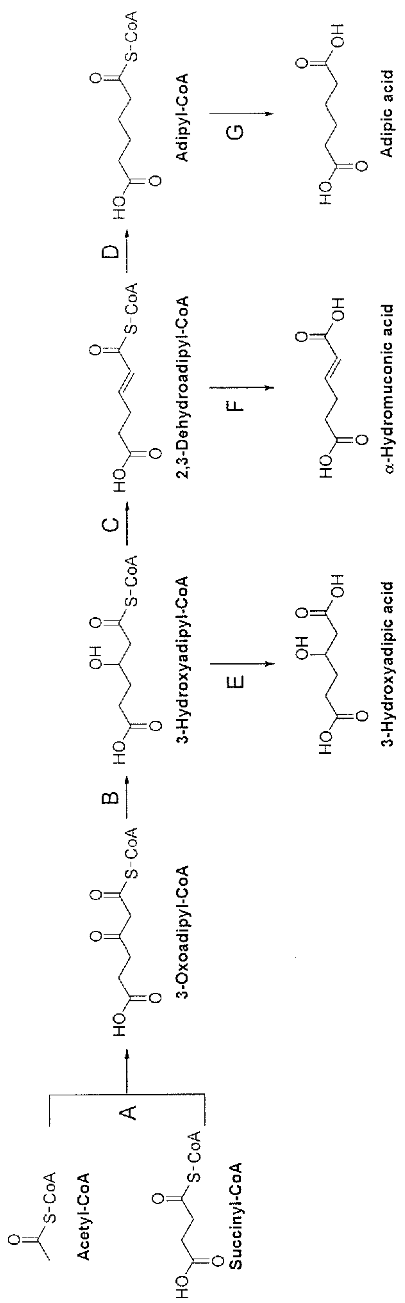

Acetyl-CoA
Succinyl-CoA
3-Oxoadipyl-CoA
3-Hydroxyadipyl-CoA
2,3-Dehydroadipyl-CoA
Adipyl-CoA
3-Hydroxyadipic acid
α-Hydromuconic acid
Adipic acid

The above Scheme 1 shows an exemplary reaction pathway required for the production of 3HA, HMA, and/or ADA. In this scheme, the reaction A is a reaction that generates 3-oxoadipyl-CoA and coenzyme A from acetyl-CoA and succinyl-CoA. The reaction B is a reaction that generates 3-hydroxyadipyl-CoA from 3-oxoadipyl-CoA. The reaction C is a reaction that generates 2,3-dehydroadipyl-CoA from 3-hydroxyadipyl-CoA. The reaction D is a reaction that generates adipyl-CoA from 2,3-dehydroadipyl-CoA. The reaction E is a reaction that generates 3HA from 3-hydroxyadipyl-CoA. The reaction F is a reaction that generates HMA from 2,3-dehydroadipyl-CoA. The reaction G is a reaction that generates ADA from adipyl-CoA.

Specific examples of the enzymes that catalyze the reactions include acyl transferase as an enzyme that catalyzes the reaction A; 3-oxoadipyl-CoA reductase as an enzyme that catalyzes the reaction B; enoyl-CoA hydratase as an enzyme that catalyzes the reaction C; enoyl-CoA reductase as an enzyme that catalyzes the reaction D; and CoA transferase as an enzyme that catalyzes the reactions E, F, and G.

A specific example of a gene encoding the enzyme that catalyzes the reaction A is acyl transferase gene, *pcaF* (NCBI Gene ID: 1041755, SEQ ID NO: 13), from *Pseudomonas putida* strain KT2440.

A specific example of a gene encoding the enzyme that catalyzes the reaction B is 3-oxoadipyl-CoA reductase gene (NCBI Gene ID: JMPQ01000047.1, SEQ ID NO: 14) from *Serratia marcescens* strain ATCC13880.

A specific example of a gene encoding the enzyme that catalyzes the reaction C is enoyl-CoA hydratase gene, *paAF* (NCBI Gene ID: 1046932, SEQ ID NO: 15), from *Pseudomonas putida* strain KT2440.

A specific example of a gene encoding the enzyme that catalyzes the reaction D is enoyl-CoA reductase gene, *dcaA* (NCBI-Protein ID: AAL09094.1, SEQ ID NO: 16) from *Acinetobacter baylyi* strain ADP1.

A specific example of a gene encoding the enzyme that catalyzes the reactions E, F, and G is a continuous sequence including the full lengths of *peal* and *pcaJ* (NCBI Gene IDs: 1046613 and 1046612, SEQ ID NOs: 17 and 18) from *Pseudomonas putida* strain KT2440. The polypeptides encoded by *pcal* and *pcaJ* together form a complex and the resulting complex catalyzes the reactions E, F, and G.

The genes encoding the enzymes that catalyze the reactions A to G can be genes naturally present in a microorganism or may be artificially introduced into a microorganism. The introduction of the genes is not limited to a specific method, and the method can be, for example, a method for integrating a gene of interest into an expression vector autonomously replicable in a microorganism and then introducing the resulting expression vector into the microorganism or a method in which the gene of interest is integrated into the genome of a microorganism.

In the present invention, the genetically modified microorganism is cultured in a culture medium, preferably in a liquid culture medium, containing a carbon source as a raw material for fermentation, which can be utilized by normal microorganisms, to produce an organic acid(s). The culture medium used contains, in addition to the carbon source available to the genetically modified microorganism, appropriate amounts of a nitrogen source and inorganic salts, and organic trace nutrients such as amino acids and vitamins as necessary. Either a complex or chemically defined medium can be used as long as the medium contains nutrients as described above.

The raw material for fermentation is a material that can be metabolized by the genetically modified microorganism. The term "metabolize" refers to the conversion of a chemical substance, which a microorganism has taken up from the extracellular environment or produced from a different chemical substance in the intracellular environment, to another chemical substance through an enzymatic reaction. Sugars can be suitably used as the carbon source. A material other than sugars may be suitable for use as long as the material can be utilized as a sole carbon source by the genetically modified microorganism for growth. Specific examples of the suitable carbon source include monosaccharides, such as glucose, fructose, galactose, mannose, xylose, and arabinose; disaccharides and polysaccharides formed by linking these monosaccharides, such as sucrose; and saccharified starch solution, molasses, and saccharified solution made from cellulose-containing biomass.

In the case of production of 3HA, HMA and/or ADA, succinic acid which is a substrate of the CoA transferase, can also be added, in addition to the above sugars, to the culture medium for efficient production of 3-hydroxyadipic acid, $\alpha$-hydromuconic acid and/or adipic acid.

The above-listed carbon sources may be used individually or in combination. When a carbon source is added to a culture medium, the concentration of the carbon source is not specifically limited but can be appropriately set depending on the type of the carbon source. Preferred concentration of a sugar is from 5 to 300 g/L, and preferred concentration of succinic acid is from 0.1 to 100 g/L.

Examples of the nitrogen source that can be used include ammonia gas, aqueous ammonia, ammonium salts, urea, nitric acid salts, and other auxiliary organic nitrogen sources, such as oil cakes, soybean hydrolysate, casein degradation products, other amino acids, vitamins, corn steep liquor, yeast or yeast extract, meat extract, peptides such as peptone, and cells of various fermentative bacteria and hydrolysate thereof. The concentration of a nitrogen source in the culture medium is not specifically limited, but a preferred concentration of the nitrogen source is from 0.1 to 50 g/L.

Examples of the inorganic salts used for culturing the genetically modified microorganism include phosphoric acid salts, magnesium salts, calcium salts, iron salts, and manganese salts, and these salts can be appropriately added to the culture medium and used.

The conditions for culturing the genetically modified microorganism to produce an organic acid(s) are set by appropriately adjusting or selecting the composition of a culture medium, a culture temperature, a stirring speed, a pH value, an aeration rate, and an inoculation amount depending on the type of the genetically modified microorganism, external conditions, and the like. In cases where foam is formed during liquid culture, an antifoaming agent such as a mineral oil, silicone oil, or surfactant may be appropriately added to the culture medium.

Once an amount of an organic acid large enough to be recovered is produced in a culture of the genetically modified microorganism, the product produced can be recovered from the culture. Recovery of the produced product, for example, isolation of the produced product, can be performed in accordance with a commonly used method, in which the incubation is stopped when the product is accumulated to an appropriate level, and the fermentation product is then recovered from the culture. Specifically, the product can be isolated from the culture by column chromatography, ion exchange chromatography, activated charcoal treatment, crystallization, membrane separation, distillation, or the like after separation of bacterial cells from the culture by centrifugation, filtration, or the like. More specifically, examples of a recovery method can include, but are not limited to, the following methods: a method in which a precipitate is collected after adding an acidic component to a salt of the product; a method in which the concentration of the product in the culture is increased by removing water from the culture in a concentration process by using a reverse osmosis membrane,

an evaporator, or the like and the concentrate is distilled to collect the product or is applied to cooling or adiabatic crystallization to precipitate the product and/or a salt of the product in crystal forms and then to recover the crystals through centri fugation, filtration, or the like; and a method in which an alcohol is added to the culture to esterify the product and the resulting ester of the product is collected by distillation and subsequently decomposed by hydrolysis to recover the product. Moreover, these recovery methods can be appropriately selected and optimized depending on the physical properties of the product, and the like.

Examples

(Reference Example 1) Preparation of Nucleic Acid Encoding Mutated YeeX Homolog of SEQ ID NO: 12 for Integration into Genome of *Serratia grimesii* (*S. grimesii*) Strain NBRC13537

For integration of a gene encoding a mutated YeeX homolog into the genome of the *S. grimesii* strain, the method using λ Red recombinase and *sacB* gene was used. A nucleic acid with a sequence required for integration of the nucleic acid into the genome was obtained by nucleic acid synthesis (manufactured by Genewiz). The sequence of the nucleic acid (SEQ ID NO: 19) contains a 840-bp region upstream of a gene encoding a homolog of the wild-type YeeX protein on the genome of the S. *grimesii* strain, *sacB* gene, a kanamycin resistance gene, and a 840-bp region downstream of the gene encoding the homolog of the wild-type YeeX protein. Primers (SEQ ID NOs: 20 and 21) for PCR amplification of the nucleic acid fragment obtained by the nucleic acid synthesis were designed, and PCR reaction was performed according to a conventional method. The obtained fragment was purified by a combination of electrophoresis using agarose gel and a column-based nucleic acid purification kit (manufactured by Cytiva), and the purified fragment was used for the production of a mutant strain.

(Reference Example 2) Preparation of Plasmid 1

The pBBR1MCS-2 vector (ME Kovach, (1995) Gene, 166: 175-176), capable of autonomous replication in *Escherichia coli* and *S. grimesii,* was cleaved with *XhoI* to obtain pBBR1MCS-2/XhoI. To integrate a constitutive expression promoter into the vector, primers (SEQ ID NOs: 23 and 24) were designed for PCR amplification of the upstream 200-bp region (SEQ ID NO: 22) of *gapA* (NCBI Gene ID: NC_000913.3) using the genomic DNA of *Escherichia coli (E. coli)* str. K-12 substr. MG1655 as a template, and PCR reaction was performed according to a conventional method. The obtained fragment and the pBBR1MCS-2/XhoI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed by routine procedures, and the plasmid was designated as pBBR1MCS-2::Pgap. Then, the pBBR1MCS-2::Pgap was cleaved with *Sca*I to obtain pBBR1MCS-2::Pgap/ScaI.

For amplification of a gene encoding an enzyme catalyzing the reaction A, primers (SEQ ID NOs: 25 and 26) were designed for PCR amplification of the full length of the acyl transferase gene *pcaF* (NCBI Gene ID: 1041755, SEQ ID NO: 13) using the genomic DNA of *Pseudomonas putida* strain KT2440 as a template, and PCR reaction was performed according to a conventional method. The obtained fragment and the pBBR1MCS-2::Pgap/ScaI were ligated together using the In-Fusion HD Cloning Kit, and the resulting plasmid was introduced into *E. coli* strain DH5a. The nucleotide sequence on the plasmid isolated from the obtained recombinant strain was confirmed by routine procedures, and the plasmid was designated as pBBR1MCS-2::AT.

Then, the pBBR1MCS-2::AT was cleaved with *Hpa*I to obtain pBBR1MCS-2::AT/HpaI. For amplification of a gene encoding an enzyme catalyzing the reactions E, F, and G, primers (SEQ ID NOs: 27 and 28) were designed for PCR amplification of a continuous sequence including the full lengths of genes together encoding a CoA transferase, *pcaI* and *pcaJ* (NCBI Gene IDs: 1046613 and 1046612, SEQ ID NOs: 17 and 18), using the genomic DNA of *Pseudomonas putida* strain KT2440 as a template, and PCR reaction was performed according to a conventional method. The obtained fragment and the pBBR1MCS-2::AT/HpaI were ligated together using the In-Fusion HD Cloning Kit, and the resulting plasmid was introduced into *E. coli* strain DH5α. The nucleotide sequence on the plasmid isolated from the obtained recombinant strain was confirmed by routine procedures, and the plasmid was designated as pBBR1MCS-2::ATCT.

The pBBR1MCS-2::ATCT was cleaved with *Seal* to obtain pBBR1MCS-2::ATCT/ScaI. For amplification of a nucleic acid encoding a 3-oxoadipyl-CoA reductase catalyzing the reaction B, primers (SEQ ID NOs: 29 and 30) were designed for PCR amplification of a nucleic acid of SEQ ID NO: 14 using the genomic DNA of *Serratia marcescens* strain ATCC13880 as a template, and PCR reaction was performed according to a conventional method. The obtained fragment and the pBBR1MCS-2::ATCT/ScaI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed by routine procedures, and the plasmid was designated as plasmid 1.

(Reference Example 3) Preparation of Plasmid 2

The pMW119 (manufactured by Nippon Gene Co., Ltd.) was cleaved with *Sac*I to obtain pMW119/SacI. To integrate a constitutive expression promoter into the vector, primers (SEQ ID NOs: 31 and 32) were designed for PCR amplification of the upstream 200-bp region (SEQ ID NO: 22) of *gapA* (NCBI Gene ID: NC_000913.3) using the genomic DNA of *E. coli* str. K-12 substr. MG1655 as a template, and PCR reaction was performed according to a conventional method. The obtained fragment and the pMW119/SacI were ligated together using the In-Fusion HD Cloning Kit (manufactured by Takara Bio Inc.), and the resulting plasmid was introduced into *E. coli* strain DH5a. The nucleotide sequence on the plasmid isolated from the obtained recombinant strain was confirmed by routine procedures, and the plasmid was designated as pMW119::Pgap.

Then, the pMW1 19::Pgap was cleaved with *Sph*I to obtain pMW119::Pgap/SphI. For amplification of a gene encoding an enzyme catalyzing the reaction C, primers (SEQ ID NOs: 33 and 34) were designed for PCR amplification of the full length of the enoyl-CoA hydratase gene *paaF* (NCBI Gene ID: 1046932, SEQ ID NO: 15) using the genomic DNA of *Pseudomonas putida* strain KT2440 as a template, and PCR reaction was performed according to a conventional method. The obtained fragment and the pMW119::Pgap/SphI were ligated together using the In-Fusion HD Cloning Kit (manufactured by Takara Bio Inc.), and the resulting plasmid was introduced into *E. coli* strain DH5$\alpha$. The nucleotide sequence on the plasmid isolated from the obtained recombinant strain was confirmed by routine procedures. The obtained plasmid was designated as pMW119::EH.

The pMW119::EH was cleaved with *Hind*III to obtain pMW119::EI-I/HindIII. For amplification of a gene encoding an enzyme catalyzing the reaction D, primers (SEQ ID NOs: 35 and 36) were designed for PCR amplification of the full length of *dcaA* (NCBI-Protein ID: AAL09094.1, SEQ ID NO: 16) from Acinetobacter *baylyi* strain ADP1, and PCR reaction was performed according to a conventional method. The obtained fragment and the pMW119::EH/HindIII were ligated together using the In-Fusion HD Cloning Kit (manufactured by Takara Bio Inc.), and the resulting plasmid was introduced into *E. coli* strain DH5$\alpha$. The nucleotide sequence on the plasmid isolated from the obtained recombinant strain was confirmed by routine procedures, and the plasmid was designated as plasmid 2.

(Reference Example 4) Preparation of Plasmid (Plasmid 3) for Expression of Mutated YeeX Homolog Represented by SEQ ID NO: 12

The pMW119::Pgap described in Reference Example 3 was cleaved with *KpnI* to obtain pMW119::Pgap/KpnI. Primers (SEQ ID NOs: 38 and 39) were designed for PCR amplification of the full length of a gene (SEQ ID NO: 37) encoding the mutated YeeX homolog of SEQ ID NO: 12, and PCR reaction was performed according to a conventional method. The obtained fragment and the pMW119::Pgap/Kpnl were ligated together using the In-Fusion HD Cloning Kit, and the resulting plasmid was introduced into *E. coli* strain DH5$\alpha$. The nucleotide sequence on the plasmid isolated from the obtained recombinant strain was confirmed by routine procedures, and the plasmid was designated as plasmid 3.

(Reference Example 5) Preparation of Nucleic Acid Encoding Mutated YeeX Represented by SEQ ID NO: 44 for Integration into Genome of *Escherichia coli* (*E. coli*) strain MG1655

For integration of a gene encoding a mutated YeeX into the genome of *E. coli* strain MG1655, the method using $\lambda$ Red recombinase and *sacB* gene was used. A nucleic acid with a sequence required for integration of the nucleic acid into the genome was obtained by nucleic acid synthesis (manufactured by Genewiz). The sequence of the nucleic acid (SEQ ID NO: 45) contains a 500-bp region upstream of the gene encoding the wild-type YeeX on the genome of the *E. coli* strain, *sacB* gene, a kanamycin resistance gene, and a 500-bp region downstream of the gene encoding the wild-type YeeX. Primers (SEQ ID NOs: 46 and 47) for PCR amplification of the nucleic acid fragment obtained by the nucleic acid synthesis were designed, and PCR reaction was performed according to a conventional method. The obtained fragment was purified by a combination of electrophoresis using agarose gel and a column-based nucleic acid purification kit (manufactured by Cytiva), and the purified fragment was used for the production of a mutant strain.

(Reference Example 6) Preparation of Plasmid for Expression of Mutated YeeX Represented by SEQ ID NO: 44 (Plasmid 4)

The pCDF-lb plasmid was cleaved with *Kpnl* to obtain pCDF-1b/KpnI. Primers (SEQ ID NOs: 49 and 50) were designed for PCR amplification of the full length of a gene encoding a mutated YeeX homolog of SEQ ID NO: 44 with the 500-bp upstream and 500-bp downstream regions of the gene (SEQ ID NO: 48), and PCR reaction was performed according to a conventional method. The obtained fragment and the pCDF-1b/KpnI were ligated together using the In-Fusion HD Cloning Kit, and the resulting plasmid was introduced into *E. coli* strain DH5$\alpha$. The nucleotide sequence on the plasmid isolated from the obtained recombinant strain was confirmed by routine procedures, and the plasmid was

designated as plasmid 4.

(Comparative Example 1) Culturing of *S. grimesii* Strain Containing Gene Encoding Protein Represented by SEQ ID NO: 2

*Serratia grimesii* strain NBRC13537 in which a gene encoding the glucose transporter PtsG (SEQ ID NO: 40) and genes encoding the pyruvate kinases PykF and PykA (SEQ ID NOs: 41 and 42) were deleted was used as a parent strain containing a homolog of the wild-type YeeX protein of SEQ ID NO: 2.

A loopful of the parent *S. grimesii* strain was inoculated into 5 mL of LB medium (10 g/L Bacto tryptone (manufactured by Difco Laboratories), 5 g/L Bacto yeast extract (manufactured by Difco Laboratories), 5 g/L sodium chloride) adjusted to pH 7 and was cultured at 30°C with shaking at 120 $min^{-1}$ for 18 hours. Subsequently, 0.15 mL of the culture fluid was added to 15 mL of the culture medium I (10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto tryptone, 1.25 g/L Bacto yeast extract) adjusted to pH 6.5 in a screw-cap test tube and was cultured at 30°C with shaking at 120 $min^{-1}$ for 48 hours.

The supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

[Conditions for Quantitative Analysis of Glucose, Acetic Acid, and Succinic Acid by HPLC]

HPLC: Shimazu Prominence (manufactured by Shimadzu Corporation)
Column: Shodex Sugar SH1011 (manufactured by Showa Denko K.K.), length: 300 mm, internal diameter: 8 mm, particle size: 6 μm
Mobile phase: 0.05M aqueous sulfuric acid solution
Flow rate: 0.6 mL/min
Column temperature: 65°CDetector: RI.

[Conditions for Quantitative Analysis of 3HA, HMA and ADA by LC-MS/MS]

HPLC: 1290 Infinity (manufactured by Agilent Technologies, Inc.)
Column: Synergi hydro-RP (manufactured by Phenomenex Inc.), length: 100 mm, internal diameter: 3 mm, particle size: 2.5 μm
Mobile phase: 0.1% aqueous formic acid solution / methanol = 70/30Flow rate: 0.3 mL/min
Column temperature: 40°C
LC detector: DAD (210 nm)
MS/MS: Triple-Quad LC/MS (manufactured by Agilent Technologies, Inc.)
Ionization method: ESI in negative mode.

(Comparative Example 2) Preparation and Culturing of *S. grimesii*/*yeeX* Deletion Strain Lacking Gene Encoding YeeX protein

The pKD46 plasmid required for expression of λ Red recombinase was introduced by electroporation into the *S. grimesii* strain described in Comparative Example 1. After the introduction, the strain was cultured at 30°C on LB agar medium containing 500 μg/mL ampicillin. The nucleic acid fragment prepared in Reference Example 1 was introduced into the obtained strain by electroporation. After the introduction, the strain was cultured at 30°C on LB agar medium containing 25 μg/mL kanamycin. The obtained recombinant strain is a *S. grimesii*/pKD46/*yeeX* deletion strain in which the full-length sequence of the gene encoding a YeeX protein homolog has been replaced with the sequence of the recombination cassette. A loopful of the *S. grimesii*/pKD46/*yeeX* deletion strain was inoculated into 5 mL of LB medium (10 g/L Bacto tryptone (manufactured by Difco Laboratories), 5 g/L Bacto yeast extract (manufactured by Difco Laboratories), 5 g/L sodium chloride) and was cultured at 37°C with shaking at 120 $min^{-1}$ for 48 hours in order to remove the pKD46 plasmid from the strain. A 10-μL aliquot of the culture fluid was incubated on LB agar medium at 30°C to select colonies lacking ampicillin resistance, from which *a S. grimesii*/*yeeX* deletion strain was obtained.

A loopful of the strain was inoculated into 5 mL of LB medium (10 g/L Bacto tryptone (manufactured by Difco

Laboratories), 5 g/L Bacto yeast extract (manufactured by Difco Laboratories), 5 g/L sodium chloride) adjusted to pH 7 and containing 25 μg/mL kanamycin and was cultured at 30°C with shaking at 120 $min^{-1}$ for 18 hours. Subsequently, 0.15 mL of the culture fluid was added to 15 mL of the culture medium I (10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto tryptone, 1.25 g/L Bacto yeast extract) adjusted to pH 6.5 and containing 25 μg/mL kanamycin in a screw-cap test tube and was cultured at 30°C with shaking at 120 $min^{-1}$ for 48 hours.

The supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

(Example 1) Preparation and Culturing of *S. grimesii*/YeeX Mutant Strain Containing Gene Encoding Mutated YeeX Homolog (SEQ ID NO: 12)

Primers of SEQ ID NOs: 20 and 21 were used to amplify the full length of a nucleic acid sequence (SEQ ID NO: 43) containing the 840-bp region upstream of the gene encoding a homolog of the wild-type YeeX protein on the genome of the *S. grimesii* strain, the gene encoding the mutated YeeX homolog of SEQ ID NO: 12, and the 840-bp region downstream of the gene encoding the homolog of the wild-type YeeX protein, and PCR reaction was performed according to a conventional method. The obtained fragment was purified by a combination of electrophoresis using agarose gel and a column-based nucleic acid purification kit (manufactured by Cytiva), and the purified fragment was used for the production of a mutant strain.

The nucleic acid fragment (SEQ ID NO: 43) was introduced by electroporation into the *S. grimesii*/*yeeX* deletion strain comprising the pKD46 plasmid, prepared in Comparative Example 2. After the introduction, the strain was cultured at 30°C on a culture medium I-based agar medium containing 50 g/L sucrose. The obtained colonies were cultured at 30°C on LB agar medium and on LB agar medium containing 25 μg/mL kanamycin to select a strain lacking kanamycin resistance. The obtained strain is a *S. grimesii*/YeeX mutant strain in which the gene encoding a homolog of YeeX protein (SEQ ID NO: 2) on the genome with no substitution of the alanine corresponding to the amino acid at position 84 in the amino acid sequence of SEQ ID NO: 1 has been replaced with the gene encoding a mutated YeeX homolog (SEQ ID NO: 12) with substitution of the alanine corresponding to the amino acid at position 84 in the amino acid sequence of SEQ ID NO: 1 to valine.

The strain was cultured in the same manner as in Comparative Example 1. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of the organic acids accumulated in the culture supernatant, the yields of the organic acids calculated using the formula (2) are shown in Table 3.

(Comparative Example 3) Preparation and Culturing of *S. grimesii* Strain Containing Plasmid 1

The plasmid 1 prepared in Reference Example 2 was introduced by electroporation into the *S. grimesii* strain described in Comparative Example 1. After the introduction, the strain was cultured at 30°C on LB agar medium containing 25 μg/mL kanamycin. The obtained recombinant strain was designated as *S. grimesii*/plasmid 1 strain.

The strain was cultured in the same manner as in Comparative Example 2. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

(Example 2) Preparation and Culturing *S. grimesii*/YeeX Mutant Strain Containing Plasmid 1

The plasmid 1 prepared in Reference Example 2 was introduced by electroporation into the *S. grimesii*/YeeX mutant strain prepared in Example 1. After the introduction, the strain was cultured at 30°C on LB agar medium containing 25 μg/mL kanamycin. The obtained recombinant strain was designated as *S. grimesiilplasmid* 1/YeeX mutant strain.

The strain was cultured in the same manner as in Comparative Example 2. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

(Example 3) Preparation and Culturing of *S. grimesii* Strain Containing Plasmid 1 and Plasmid 3

The plasmid 3 prepared in Reference Example 4 was introduced by electroporation into the *S. grimesii*/plasmid 1 strain prepared in Comparative Example 3. After the introduction, the strain was cultured at 30°C on LB agar medium containing 25 μg/mL kanamycin and 500 μg/mL ampicillin. The obtained recombinant strain was designated as *S. grimesii*/*plasmid* 1/plasmid 3 strain.

A loopful of the strain was inoculated into 5 mL of LB medium (10 g/L Bacto tryptone (manufactured by Difco Laboratories), 5 g/L Bacto yeast extract (manufactured by Difco Laboratories), 5 g/L sodium chloride) adjusted to pH 7 and containing 25 μg/mL kanamycin and 500 μg/mL ampicillin and was cultured at 30°C with shaking at 120 $min^{-1}$ for 18 hours. Subsequently, 0.15 mL of the culture fluid was add to 15 mL of the culture medium I (10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto tryptone, 1.25 g/L Bacto yeast extract) adjusted to pH 6.5 and containing 25 μg/mL kanamycin and 500 μg/mL ampicillin in a screw-cap test tube and was cultured at 30°C with shaking at 120 $min^{-1}$ for 48 hours.

The supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

(Comparative Example 4) Preparation and Culturing of *S. grimesii* Strain Containing Plasmid 1 and Plasmid 2

The plasmid 2 prepared in Reference Example 3 was introduced by electroporation into the *S. grimesii*/plasmid 1 strain prepared in Comparative Example 3. After the introduction, the strain was cultured at 30°C on LB agar medium containing 25 μg/mL kanamycin and 500 μg/mL ampicillin. The obtained recombinant strain was designated as *S. grimesiilplasmid* 1/plasmid 2 strain.

The strain was cultured in the same manner as in Example 3. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

(Example 4) Preparation and Culturing *S. grimesii*/YeeX Mutant Strain Containing Plasmid 1 and Plasmid 2

The plasmid 2 prepared in Reference Example 3 was introduced by electroporation into the *S. grimesii*/YeeX mutant strain prepared in Example 1. After the introduction, the strain was cultured at 30°C on LB agar medium containing 25 μg/mL kanamycin and 500 μg/mL ampicillin. The obtained recombinant strain was designated as *S. grimesii*/plasmid 1/plasmid 2/YeeX mutant strain.

The strain was cultured in the same manner as in Example 3. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 3.

[Table 3]

| Comparative Example Example | S.grimesii strain | Plasmid | succinic acid (%) | acetic acid (%) | 3HA (%) | HMA (%) | ADA (%) |
|---|---|---|---|---|---|---|---|
| Comparative Example 1 | parent strain | none | 65 | 39 | - | - | - |
| Comparative Example 2 | YeeX deletion strain | none | 60 | 34 | - | - | - |
| Example 1 | YeeX mutant strain | none | 106 | 81 | - | - | - |
| Comparative Example 3 | parent strain | 1 | 60 | 33 | 4.3 | 0.02 | - |
| Example 2 | YeeX mutant strain | 1 | 90 | 71 | 5.4 | 0.16 | - |

(continued)

| Comparative Example Example | S.grimesii strain | Plasmid | succinic acid (%) | acetic acid (%) | 3HA (%) | HMA (%) | ADA (%) |
|---|---|---|---|---|---|---|---|
| Example 3 | parent strain | 1,3 | 64 | 35 | 4.6 | 0.20 | - |
| Comparative Example 4 | parent strain | 1,2 | 57 | 33 | 3.4 | 0.21 | 0.07 |
| Example 4 | YeeX mutant strain | 1, 2 | 93 | 58 | 7.5 | 0.55 | 0.12 |

The results of Comparative Examples 1 and 2 and Example 1 indicated that the S. *grimesii*/YeeX mutant strain comprising the gene encoding a mutated YeeX homolog (SEQ ID NO: 12) on the genome produced higher yields of the organic acids such as succinic acid and acetic acid than the parent *S. grimesii* strain comprising the gene encoding the homolog of the wild-type YeeX protein (SEQ ID NO: 2) on the genome or than the *S. grimesii*/YeeX deletion strain.

The results of Comparative Example 3 and Example 2 indicated that the yields of the organic acids such as succinic acid, acetic acid, 3HA, and HMA produced by fermentation were also increased in the *S. grimesii*/plasmid 1/YeeX mutant strain comprising the plasmid 1 for expression of an enzyme required for production of 3HA compared with the parent strain comprising the plasmid 1.

In Example 3, the yields of the organic acids were found to be also higher in the *S. grimesiilplasmid* 1/plasmid 3 strain comprising the gene encoding the homolog of the wild-type YeeX protein (SEQ ID NO: 2) on the genome and further comprising the plasmid 1 and the plasmid 3 for expression of a mutated YeeX homolog (SEQ ID NO: 12). This indicated that the effect of the present invention is also provided in the *S. grimesii* strain expressing both the homolog of the wild-type YeeX protein and the mutated YeeX homolog by the expression plasmid used to introduce the mutated YeeX homolog.

The results of Comparative Example 4 and Example 4 indicated that the yields of the organic acids such as succinic acid, acetic acid, 3HA, HMA, and ADA was also remarkably increased in the *S. grimesii*/*plasmid* 1/plasmid 2/YeeX mutant strain comprising the plasmid 1 and plasmid 2 for expression of enzymes required for the production of ADA compared with the parent strain comprising the plasmid 1 and plasmid 2.

(Comparative Example 5) Culturing of *E. coli* Strain Containing Gene Encoding Wild-Type YeeX Protein (SEQ ID NO: 1)

The Escherichia *coli* strain MG1655 was used as a parent strain containing the wild-type YeeX protein of SEQ ID NO: 1. The strain was cultured in the same manner as in Comparative Example 1. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Comparative Example 6) Preparation and Culturing *of E. colilyeeX* Deletion Strain Lacking Gene Encoding YeeX protein

The pKD46 plasmid required for expression of λ Red recombinase was introduced by electroporation into the *E. coli* strain described in Comparative Example 5. After the introduction, the strain was cultured at 30°C on LB agar medium containing 50 μg/mL ampicillin. The nucleic acid fragment prepared in Reference Example 5 was introduced into the obtained strain by electroporation. After the introduction, the strain was cultured at 30°C on LB agar medium containing 25 μg/mL kanamycin. The obtained recombinant strain is an *E. coli*/pKD46/*yeeX* deletion strain in which the full-length sequence of the gene encoding YeeX has been replaced with the sequence of the recombination cassette. A loopful of the *E. coli*/*pKD46*/*yeeX* deletion strain was inoculated into 5 mL of LB medium (10 g/L Bacto tryptone (manufactured by Difco Laboratories), 5 g/L Bacto yeast extract (manufactured by Difco Laboratories), 5 g/L sodium chloride) and was cultured at 37°C with shaking at 120 $min^{-1}$ for 48 hours to remove the pKD46 plasmid from the strain. A 10-μL aliquot of the culture fluid was incubated on LB agar medium at 30°C to select colonies lacking ampicillin resistance, from which an *E. colilyeex* deletion strain was obtained.

The strain was cultured in the same manner as in Comparative Example 2. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Example 5) Preparation and Culturing of *E. coli*/YeeX Mutant Strain Containing Gene Encoding Mutated YeeX Protein (SEQ ID NO: 44)

Primers of SEQ ID NOs: 46 and 47 were used to amplify the full length of a nucleic acid sequence (SEQ ID NO: 48) containing a 500-bp region upstream of the gene encoding the wild-type YeeX protein (SEQ ID NO: 1) on the genome of the *E. coli* strain, the gene encoding the mutated YeeX protein of SEQ ID NO: 44, and a 500-bp region downstream of the gene encoding the wild-type YeeX protein, and PCR reaction was performed according to a conventional method. The obtained fragment was purified by a combination of electrophoresis using agarose gel and a column-based nucleic acid purification kit (manufactured by Cytiva), and the purified fragment was used for the production of a mutant strain.

The nucleic acid fragment (SEQ ID NO: 48) was introduced by electroporation into the *E. coli*/*yeeX* deletion strain comprising the pKD46 plasmid, prepared in Comparative Example 6. After the introduction, the strain was cultured at 30°C on a culture medium I-based agar medium containing 50 g/L sucrose. The obtained colonies were cultured at 30°C on LB agar medium and on LB agar medium containing 25 μg/mL kanamycin to select a strain lacking kanamycin resistance. The obtained strain is an E. coli/YeeX mutant strain in which the gene encoding the wild-type YeeX protein of SEQ ID NO: 1 on the genome has been replaced with the gene encoding a mutated YeeX protein (SEQ ID NO: 44) with substitution of the alanine corresponding to the amino acid at position 84 in the amino acid sequence of SEQ ID NO: 1 to valine.

The strain was cultured in the same manner as in Comparative Example 1. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Comparative Example 7) Preparation and Culturing of *E.* coli Strain Containing Plasmid 1

The plasmid 1 prepared in Reference Example 2 was introduced by electroporation into the *E. coli* strain described in Comparative Example 5. After the introduction, the strain was cultured at 30°C on LB agar medium containing 25 μg/mL kanamycin. The obtained recombinant strain was designated as *E. coli*/plasmid 1 strain.

The strain was cultured in the same manner as in Comparative Example 2. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Example 6) Preparation and Culturing of *E. coli*/*YeeX* Mutant Strain Containing Plasmid 1

The plasmid 1 prepared in Reference Example 2 was introduced by electroporation into the *E.* coli/YeeX mutant strain prepared in Example 5. After the introduction, the strain was cultured at 30°C on LB agar medium containing 25 μg/mL kanamycin. The obtained recombinant strain was designated as E. *coli*/plasmid 1/YeeX mutant strain.

The strain was cultured in the same manner as in Comparative Example 2. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Example 7) Preparation and Culturing of *E. coli* Strain Containing Plasmid 1 and Plasmid 4

The plasmid 4 prepared in Reference Example 6 was introduced by electroporation into the *E. coli*/plasmid 1 strain prepared in Comparative Example 7. After the introduction, the strain was cultured at 30°C on LB agar medium containing 25 μg/mL kanamycin and 50 μg/mL streptomycin. The obtained recombinant strain was designated as E. coli/plasmid 1/plasmid 4 strain.

A loopful of the strain was inoculated into 5 mL of LB medium (10 g/L Bacto tryptone (manufactured by Difco Laboratories), 5 g/L Bacto yeast extract (manufactured by Difco Laboratories), 5 g/L sodium chloride) adjusted to pH 7 and containing 25 μg/mL kanamycin and 50 μg/mL streptomycin and was cultured at 30°C with shaking at 120 $min^{-1}$ for 18 hours. Subsequently, 0.15 mL of the culture fluid was added to 15 mL of the culture medium I (10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto tryptone, 1.25 g/L Bacto yeast

extract) adjusted to pH 6.5 and containing 25 μg/mL kanamycin and 50 μg/mL streptomycin in a screw-cap test tube and was cultured at 30°C with shaking at 120 $min^{-1}$ for 48 hours.

The supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Comparative Example 8) Preparation and Culturing of *E. coli* Strain Containing Plasmids 1 and 2

The plasmid 2 prepared in Reference Example 3 was introduced by electroporation into the *E. coli*/plasmid 1 strain prepared in Comparative Example 7. After the introduction, the strain was cultured at 30°C on LB agar medium containing 25 μg/mL kanamycin and 50 μg/mL ampicillin. The obtained recombinant strain was designated as *E. coli*/plasmid 1/plasmid 2 strain.

A loopful of the strain was inoculated into 5 mL of LB medium (10 g/L Bacto tryptone (manufactured by Difco Laboratories), 5 g/L Bacto yeast extract (manufactured by Difco Laboratories), 5 g/L sodium chloride) adjusted to pH 7 and containing 25 μg/mL kanamycin and 50 μg/mL ampicillin and was cultured at 30°C with shaking at 120 $min^{-1}$ for 18 hours. Subsequently, 0.15 mL of the culture fluid was added to 15 mL of the culture medium I (10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto tryptone, 1.25 g/L Bacto yeast extract) adjusted to pH 6.5 and containing 25 μg/mL kanamycin and 50 μg/mL ampicillin in a screw-cap test tube and was cultured at 30°C with shaking at 120 $min^{-1}$ for 48 hours.

The supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

(Example 8) Preparation and Culturing of *E. coli*/YeeX Mutant Strain Containing Plasmid 1 and Plasmid 2

The plasmid 2 prepared in Reference Example 3 was introduced by electroporation into the *E. cali*/*NeeX* mutant strain prepared in Example 5. After the introduction, the strain was cultured at 30°C on LB agar medium containing 25 μg/mL kanamycin and 50 μg/mL ampicillin. The obtained recombinant strain was designated as *E. coli*/plasmid 1/plasmid 2/YeeX mutant strain.

The strain was cultured in the same manner as in Comparative Example 8. Subsequently, the supernatant separated from bacterial cells by centrifugation of the culture fluid was filtered through a Millex-GV membrane (0.22 μm; PVDF; manufactured by Merck), and the resulting filtrate was analyzed by HPLC and LC-MS/MS. As the result of the quantitative analysis of organic acids accumulated in the culture supernatant, the yields of the organic acids calculated using the formula (2) are shown in Table 4.

[Table 4]

| Comparative Example Example | *E. coli* MG1655 strain | plasmid | succinic acid (%) | acetic acid (%) | 3HA (%) | HMA (%) | ADA (%) |
|---|---|---|---|---|---|---|---|
| Comparative Example 5 | parent strain | none | 19.3 | 33.4 | - | - | - |
| Comparative Example 6 | YeeX deletion strain | none | 18.4 | 31.0 | - | - | - |
| Example 5 | YeeX mutant strain | none | 30.6 | 55.0 | - | - | - |
| Comparative Example 7 | parent strain | 1 | 15.2 | 27.0 | 1.7 | - | - |
| Example 6 | YeeX mutant strain | 1 | 24.6 | 46.7 | 2.9 | - | - |
| Example 7 | parent strain | 1, 4 | 34.2 | 55.7 | 2.9 | - | - |
| Comparative Example 8 | parent strain | 1, 2 | 14.0 | 25.1 | 1.2 | 0.021 | 0.030 |
| Example 8 | YeeX mutant strain | 1, 2 | 22.5 | 41.0 | 1.9 | 0.032 | 0.044 |

The results of Comparative Examples 5 and 6 and Example 5 indicated that the *E. coli*/YeeX mutant strain comprising the gene encoding a mutated YeeX protein (SEQ ID NO: 44) on the genome produced higher yields of the organic acids such as succinic acid and acetic acid than the parent *E. coli* strain comprising the gene encoding the wild-type YeeX protein (SEQ ID NO: 1) on the genome or than the *E. coli*/YeeX deletion strain.

The results of Comparative Example 7 and Example 6 indicated that the yields of the organic acids such as succinic acid, acetic acid, and 3HA produced by fermentation were also increased in the *E. coli*/plasmid 1/YeeX mutant strain comprising the plasmid 1 for expression of an enzyme required for the production of 3HA compared with the parent strain comprising the plasmid 1.

In Example 6, the yields of the organic acids were found to be also higher in the *E*. coli/plasmid 1/plasmid 4 strain comprising the gene encoding the wild-type YeeX protein (SEQ ID NO: 1) on the genome and further comprising the plasmid 1 and the plasmid 4 for expression of a mutated YeeX protein (SEQ ID NO: 44). This indicated that the effect of the present invention is also provided in the *E. coli* strain expressing both the wild-type YeeX protein and the mutated YeeX protein by the expression plasmid used to introduce the mutated YeeX protein.

The results of Comparative Example 8 and Example 8 indicated that the yields of the organic acids such as succinic acid, acetic acid, 3HA, HMA, and ADA was also remarkably increased in the *E. coli*/plasmid 1/plasmid 2/YeeX mutant strain comprising the plasmids 1 and 2 for expression of enzymes required for the production of ADA compared with the parent strain comprising the plasmids 1 and 2.

## Claims

1. A genetically modified microorganism comprising a gene capable of expressing a mutated YeeX protein or a homolog thereof, which results from substitution, insertion, and/or deletion of one to several amino acids in the amino acid sequence of the wild-type YeeX protein or of a homolog thereof.

2. The genetically modified microorganism according to claim 1, wherein said mutated YeeX protein or a homolog thereof is a mutated YeeX protein or a homolog thereof, which results from substitution, insertion, and/or deletion of one to several amino acids within a region corresponding to the amino acid residues 74 to 100 in the amino acid sequence of SEQ ID NO: 1.

3. The genetically modified microorganism according to claim 1 or 2, wherein said mutated YeeX protein or a homolog thereof is a mutated YeeX protein or a homolog thereof with a mutation, which is a substitution of the alanine corresponding to the amino acid at position 84 in the amino acid sequence of SEQ ID NO: 1 to another amino acid.

4. The genetically modified microorganism according to any one of claims 1 to 3, wherein said gene capable of expressing the mutated YeeX protein or a homolog thereof is present in the genome.

5. The genetically modified microorganism according to claim 4, which results from mutation or replacement of a gene expressing the wild-type YeeX protein or a homolog thereof in the genome into or with said gene capable of expressing the mutated YeeX protein or a homolog thereof.

6. The genetically modified microorganism according to any one of claims 1 to 5, wherein said mutated YeeX protein or a homolog thereof has a mutation, which is a substitution of the alanine corresponding to the amino acid at position 84 in the amino acid sequence of SEQ ID NO: 1 to valine.

7. The genetically modified microorganism according to any one of claims 1 to 6, wherein said microorganism has an ability to produce an organic acid.

8. The genetically modified microorganism according to any one of claims 1 to 7, wherein said microorganism is a microorganism belonging to the genus *Serratia, Escherichia, Actinobacillus, Basfia, Pseudomonas, Hafnia, Acinetobacter, Shinzwellia,* or *Aerobacter.*

9. A method of producing an organic acid(s), comprising culturing said genetically modified microorganism according to claim 7 or 8 in a culture medium containing a carbon source as a raw material for fermentation.

10. The method of producing an organic acid(s) according to claim 9, wherein said organic acid(s) is/are succinic acid, acetic acid, 3-hydroxyadipic acid, $\alpha$-hydromuconic acid, and/or adipic acid.

**INTERNATIONAL SEARCH REPORT**

International application No.
PCT/JP2021/017802

A. CLASSIFICATION OF SUBJECT MATTER
Int.Cl. C12N1/21(2006.01)i, C12N15/31(2006.01)i, C12P7/40(2006.01)i, C12P7/42(2006.01)i, C12P7/44(2006.01)i, C12P7/46(2006.01)i, C12P7/54(2006.01)i
FI: C12N1/21ZNA, C12P7/40, C12P7/54, C12P7/42, C12P7/46, C12P7/44, C12N15/31
According to International Patent Classification (IPC) or to both national classification and IPC

B. FIELDS SEARCHED
Minimum documentation searched (classification system followed by classification symbols)
Int.Cl. C12N1/00-7/08, C12N15/00-15/90, C12P7/40, C12P7/00-7/66

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII), CAplus/MEDLINE/EMBASE/BIOSIS(STN), GenBank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X<br>Y<br>A | GenBank [online], ACCESSION: WP_147690482, URL: <https://www.ncbi.nlm.nih.gov/protein/WP_147690482.1?report=genbank&log$=protalign&blast_rank=60&RID=E4TRORK6016>, 29 August 2019, [retrieved on 16 July 2021], DEFINITION: DUF496 family protein [Escherichia coli], particularly, origin column, source column | 1, 2, 4, 5, 7, 8<br>9, 10<br>3, 6 |
| X<br>Y<br>A | GenBank [online], ACCESSION: WP_129485903, URL: <https://www.ncbi.nlm.nih.gov/protein/WP_129485903.1?report=genbank&log$=protalign&blast_rank=79&RID=E4TRORK6016>, 03 October 2019, [retrieved on 16 July 2021], DEFINITION: DUF496 family protein [Escherichia coli], particularly, origin column, source column | 1, 2, 4, 5, 7, 8<br>9, 10<br>3, 6 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 July 2021 | 03 August 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | <br>Telephone No. |

Form PCT/ISA/210 (second sheet) (January 2015)

**INTERNATIONAL SEARCH REPORT**

International application No. PCT/JP2021/017802

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | GenBank [online], ACCESSION: HAH7807653, URL: <https://www.ncbi.nlm.nih.gov/protein/HAH7807653.1?report=genbank&log$=protalign&blast_rank=97&RID=E4TRORK6016>, 22 April 2020, [retrieved on 16 July 2021], DEFINITION: TPA: DUF496 family protein [Escherichia coli], particularly, origin column, source column | 1, 2, 4, 5, 7, 8<br>9, 10<br>3, 6 |
| Y<br>A | WO 2019/107516 A1 (TORAY INDUSTRIES, INC.) 06 June 2019 (2019-06-06), claims 4, 8-10, paragraphs [0014], [0039], [0072], examples 1-4, 7, 8 | 9, 10<br>1-8 |

Form PCT/ISA/210 (second sheet) (January 2015)

**INTERNATIONAL SEARCH REPORT**
Information on patent family members

International application No.
PCT/JP2021/017802

| | | |
|---|---|---|
| WO 2019/107516 A1 | 06 June 2019 | CN 111386339 A<br>CA 3083563 A1<br>claims 4, 8-10,<br>paragraphs [0014], [0039], [0072],<br>examples 1-4, 7, 8 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017192325 A
- WO 2019107516 A
- US 20070298418 A


### Non-patent literature cited in the description


- *FEMS Microbiology Letters,* 1998, vol. 169, 375-382
- *Biosci. Biotechnol. Biochem.,* 2007, vol. 71 (12), 2905-2911
- *Proc. Natl. Acad. Sci. U.S.A.,* 2000, vol. 97 (12), 6640-6645
- *J. Bacteriol.,* 1988, vol. 170, 2796-2801
- *J. Mol. Biol.,* 1970, vol. 53 (1), 159-162
- **ME KOVACH.** *Gene,* 1995, vol. 166, 175-176